# EUROPÄISCHE PATENTSCHRIFT

(11) **EP 3 578 224 B1**
(45) Veröffentlichungstag und Bekanntmachung des Hinweises auf die Patenterteilung: **30.09.2020**
(21) Anmeldenummer: 18176312.9
(22) Anmeldetag: 06.06.2018
(51) Int. Cl.: A61M 37/00

(54) **NADELMODUL FÜR EINE HAUTSTECHVORRICHTUNG ZUM LOKALEN AUFSTECHEN EINER MENSCHLICHEN ODER TIERISCHEN HAUT IN SCHRÄGRICHTUNG UND HAUTSTECHVORRICHTUNG**
DEVICE AND NEEDLE MODULE FOR A DEVICE FOR LOCAL PUNCTURING OF HUMAN OR ANIMAL SKIN IN AN INCLINED DIRECTION
MODULE D'AIGUILLE POUR UN DISPOSITIF DE SUTURE DE LA PEAU DESTINÉ AU PERÇAGE LOCAL D'UNE PEAU HUMAINE OU ANIMALE DANS LE SENS OBLIQUE ET DISPOSITIF DE SUTURE DE LA PEAU

(43) Veröffentlichungstag der Anmeldung: 11.12.2019
(73) Patentinhaber: MT.DERM GmbH, 14167 Berlin (DE)
(72) Erfinder: Scherkowski, Dirk, 12489 Berlin (DE)
(74) Vertreter: Bittner, Thomas L.

(56) Entgegenhaltungen:
- EP-A1- 1 576 982
- EP-A1- 3 064 251
- CN-Y- 2 275 851
- CN-Y- 2 277 244
- KR-A- 20160 124 015

## Beschreibung

Die Erfindung betrifft ein Nadelmodul für eine Hautstechvorrichtung zum lokalen Aufstechen einer menschlichen oder tierischen Haut in Schrägrichtung sowie einer Hautstechvorrichtung.

### Hintergrund

Derartige Hautstechvorrichtungen werden genutzt, um die Haut lokal aufzustechen. Hierbei kann vorgesehen sein, mittels des lokalen Aufstechens der Haut eine Substanz in die Haut einzutragen, beispielsweise einen Farbstoff, in Verbindung mit dem Ausbilden von Tattoos oder Permanentmakeup, oder einen kosmetischen oder medizinischen Stoff.

Übliche Hautstechvorrichtungen sind als Handgerät ausgebildet, bei denen ein Antriebsmodul und ein hieran koppelndes Nadelmodul vorgesehen sind. Das Antriebsmodul weist zum Beispiel einen Elektromotor auf, mit dem eine Antriebsbewegung bereitgestellt wird, um eine Stechnadel durch eine vorderseitige Gehäuseöffnung des Nadelmoduls aus- und einzufahren. Die von dem Antriebsmodul bereitgestellte Antriebsbewegung wird auf die Stechnadel eingekoppelt, sodass diese zum lokalen Aufstechen der Haut vor- und zurückgefahren werden kann. Antriebsmodul und Nadelmodul können lösbar miteinander gekoppelt sein, wobei das Nadelmodul als ein Einwegmodul ausgeführt sein kann. Das Vor- und Zurückbewegen der Stechnadel findet im Betrieb mit einer maschinengesteuerten Wiederholfrequenz statt. EP1576982A1 offenbart ein Nadelmodul mit einem Nadelkanal, eine Stechnadel, wobei der Nadelkanal abgeknickt, aber kein Ausweichraum vorhergesehen ist. EP3064251 A1, CN2277244Y, CN2275851 Y und KR20160124015A offenbaren Nadelmodule mit flexiblen Stechnadeln, jedoch keine abgeknickten Nadelkanäle und keine Ausweichräume.

### Zusammenfassung

Aufgabe der Erfindung ist es, ein Nadelmodul für eine Hautstechvorrichtung zum lokalen Aufstechen einer menschlichen oder tierischen Haut in Schrägrichtung sowie eine Hautstechvorrichtung anzugeben, mit denen verbesserte Betriebseigenschaften bereitgestellt sind.

Zur Lösung sind ein Nadelmodul für eine Hautstechvorrichtung zum lokalen Aufstechen einer menschlichen oder tierischen Haut in Schrägrichtung sowie eine Hautstechvorrichtung nach den unabhängigen Ansprüchen 1 und 14 geschaffen. Ausgestaltungen sind Gegenstand von abhängigen Unteransprüchen.

Nach einem Aspekt ist ein Nadelmodul für eine Hautstechvorrichtung zum lokalen Aufstechen einer menschlichen oder tierischen Haut in Schrägrichtung geschaffen, wobei das Nadelmodul Folgendes aufweist: ein Gehäuse; eine Nadelaustrittsöffnung, die an dem Gehäuse frontseitig angeordnet ist; eine Nadeleinführöffnung, die an dem Gehäuse rückseitig angeordnet ist; ein Nadelkanal, welcher sich in dem Gehäuse zwischen der Nadeleinführöffnung und der Nadelaustrittsöffnung erstreckt und eingerichtet ist, eine flexible Stechnadel nadelführend aufzunehmen; einen in Bezug auf die Nadelaustrittsöffnung proximalen Kanalabschnitt des Nadelkanals, welcher sich rückseitig der Nadelaustrittsöffnung erstreckt und in welchem eine die flexible Stechnadel zur Nadelaustrittsöffnung hin führende proximale Nadelführung gebildet ist; und einen in Bezug auf die Nadelaustrittsöffnung distalen Kanalabschnitt des Nadelkanals, welcher sich rückseitig des proximalen Kanalabschnitts erstreckt und in welchem der Nadelkanal abgeknickt oder abgewinkelt ist. Der Nadelkanal weist in einem Knickbereich des distalen Kanalabschnitts eine Kanalerweiterung auf, bei der der Nadelkanal im Knickbereich mit einem Kanalquerschnitt gebildet ist, welcher größer ist als ein Kanalquerschnitt in dem proximalen Kanalabschnitt, derart, dass ein Ausweichraum für die flexible Stechnadel bereitgestellt ist, in welchen hinein die flexible Stechnadel aufgrund eines Einstechwiderstands mittels Abknicken ausweichen kann.

Nach einem weiteren Aspekt ist eine Hautstechvorrichtung zum lokalen Aufstechen einer menschlichen oder tierischen Haut in Schrägrichtung geschaffen, wobei die Hautstechvorrichtung ein Nadelmodul, eine flexible Stechnadel, die in dem Nadelmodul aus- und einfahrbar angeordnet ist, sowie ein Antriebsmodul aufweist, welches an das Nadelmodul koppelt, derart, dass eine von dem Antriebsmodul bereitgestellte Antriebsbewegung zum Ein- und Ausfahren auf die flexible Stechnadel einkoppelbar ist.

Die vorgeschlagene Ausgestaltung des Nadelkanals ermöglicht es der Stechnadel, abzuknicken und mit einem abgeknickten Abschnitt der Stechnadel in den Ausweichraum auszuweichen, wenn der Einstechwiderstand beim Aufstechen der Haut ein solches Abknicken der flexiblen Stechnadel bewirkt. Der Stechvorgang kann auf diese Weise "abgefedert werden". Die Nadelaustrittsöffnung kann bei dem Gehäuse im Bereich einer Gehäusespitze angeordnet sein, die wahlweise einen sich zur Nadelaustrittsöffnung hin verjüngenden Gehäuseabschnitt aufweist.

Der Ausweichraum kann auf einer Außenseite des Knickbereichs ausgebildet sein. Bei dieser Ausführungsform kann sich die Kanalerweiterung im Wesentlichen einseitig erstrecken, nämlich auf der Außenseite des Knickbereichs des Nadelkanals. Es kann vorgesehen sein, dass der Nadelkanal rückseitig des proximalen Kanalabschnitts sich zunächst im Wesentlichen nicht erweiternd in den distalen Kanalabschnitt fortsetzt, wobei die Kanalerweiterung dann in einem weiter hinten liegenden Bereich des distalen Kanalabschnitts gebildet ist.

Der Kanalquerschnitt kann in dem distalen Kanalabschnitt, ausgehend von dem proximalen Kanalabschnitt, sich zunehmend erweiternd ausgeführt sein. Die zunehmende Erweiterung kann beispielsweise mit einer Trichterform gebildet sein.

Zwischen einer Führrichtung für die flexible Stechnadel in dem proximalen Kanalabschnitt und der Flächennormalen auf einer Öffnungsfläche der Nadeleinführöffnung kann ein Winkel zwischen etwa 15 Grad und etwa 90 Grad, alternativ zwischen etwa 30 Grad und etwa 60 Grad, ausgebildet sein. Es ist dem Benutzer so erleichtert, beim lokalen Aufstechen der Haut mit einem solchen Winkel schräg bis flach in die Hautoberfläche einzustechen.

Das Gehäuse kann im Bereich des Ausweichraums einen den Ausweichraum erweiternden Durchbruch aufweisen. Im Betrieb kann die Stechnadel mit ihrem Knickbereich bis in den Durchbruch hinein ausweichen, wenn der Einstechwiderstand besonders groß ist. Alternativ kann der Ausweichraum im Wesentlichen vollständig mittels des Gehäusedurchbruchs bereitgestellt sein.

Die Kanalerweiterung kann zwischen einem rückseitigen Ausgang des proximalen Kanalabschnitts und der Nadeleinführöffnung den Nadelkanal mindestens teilweise erfassend gebildet sein. Die Kanalerweiterung kann mit einem im Wesentlichen gleichbleibenden oder sich ändernden Kanalquerschnitt längs des Nadelkanals ausgebildet sein.

An dem Gehäuse, zum Beispiel an der Nadelmodulspitze, kann eine Auflagefläche angeordnet sein, die zur Öffnungsfläche der Nadelaustrittsöffnung schräg gestellt ist. Die Auflagefläche erleichtert dem Benutzer die manuelle Führung der Hautstechvorrichtung mit dem Nadelmodul beim Arbeiten, um in Schrägrichtung einstechen zu können.
Die Auflagefläche kann im Wesentlichen parallel zur Öffnungsfläche der Nadeleinführöffnung angeordnet sein.

Im Knickbereich des Nadelkanals kann eine Einstelleinrichtung angeordnet sein, mit der ein Ausweichraum für das Ausweichen, ein des flexiblen Stechnadel zur Verfügung stehender

Raum oder Bereich veränderbar ist. Die Einstellung kann eingerichtet sein, den Ausweichraum stufenlos oder in festgelegten Stufen zu vergrößern und zu verkleinern. Beispielsweise kann in einer Ausgestaltung am Gehäuse eine Einstellschraube vorgesehen sein, die von außen mittels Drehen betätigbar ist, sei es manuell oder mit einem Werkzeug. Hierdurch kann ein im Ausweichraum angeordneter Anschlag, der an die Einstellschraube koppelt, zur Mitte des Nadelkanals hin und hiervon weg verstellt oder verlagert werden, um den Ausweichraum anwendungsabhängig zu dimensionieren.

Die Einstelleinrichtung kann eingerichtet sein, den für das Ausweichen der flexiblen Stechnadel zur Verfügung stehenden Raum auf der Außenseite und / oder der Innenseite des Knickbereichs des Nadelkanals einzustellen.

Auf der Innenseite des Knickbereichs des Nadelkanals kann ein Führungselement angeordnet sein, welches eingerichtet ist, die flexible Stechnadel beim Einziehen in den Nadelkanal lokal zu führen. Das Führungselement kann beispielsweise eine der Stechnadel zugewandte gekrümmte oder gerundete Oberfläche aufweisen, auf welcher die Stechnadel beim Einziehen möglichst reibungsfrei entlang gleitet und so gestützt und geführt wird.

Der Nadelkanal kann zumindest in dem proximalen Kanalabschnitt vorstehende Wandabschnitte, an denen die flexible Stechnadel im Betrieb zur Anlage kommt, und zurückstehende Wandabschnitte aufweisen, an denen die flexible Stechnadel im Betrieb nicht zur Anlage kommt. Auf diese Weise wird die Reibung für die Stechnadel an der Innenwand des Nadelkanals gemindert. Im Betrieb, also beim Ein- und Ausfahren der Stechnadel, kommt diese potenziell mit den vorstehenden Wandabschnitten in Kontakt, wohingegen ein Kontakt mit den zurückstehenden Wandabschnitten durch benachbarte vorstehende Wandabschnitte verhindert ist. Die vorstehenden Wandabschnitte können mit einer gekrümmten oder gerundeten Oberfläche gebildet sein, um Reibungswiderstand weiter zu mindern. Auch eine punkt- oder linienförmig ausgebildete Anlagefläche an den vorstehenden Wandabschnitten kann vorgesehen sein.

Die vorstehenden und die zurückstehenden Wandabschnitte können an einer Kanalwand mit Wellenoberflächenform gebildet sein.

In Verbindung mit der Hautstechvorrichtung, die das Nadelmodul und das Antriebsmodul aufweist, können die vorangehend im Zusammenhang mit dem Nadelmodul erläuterten Ausgestaltungen entsprechend vorgesehen sein.

Die Hautstechvorrichtung kann als eine maschinengesteuerte oder -angetriebene Vorrichtung ausgeführt sein, bei der eine Antriebseinrichtung im Antriebsmodul eine nicht manuell, sondern maschinell oder automatisiert erzeugte Antriebsbewegung zur Verfügung stellt. Hierbei kann die Antriebseinrichtung beispielsweise einen elektrischen Motor aufweisen.

Die flexible Stechnadel kann als Einzelnadel oder Nadelgruppe ausgeführt sein. Hierbei ist die Stechnadel insbesondere dahingehend flexibel, dass der Nadelkörper sich elastisch verbiegen kann, insbesondere in einem Knickbereich abknicken kann.

Die Wiederholfrequenz, mit der die Antriebsbewegung bereitgestellt ist, kann, im Fall der maschinengesteuerten oder -angetriebenen Vorrichtung, zwischen etwa 0,5 und etwa 200 Hz, alternativ zwischen etwa 10 und etwa 200 Hz oder zwischen etwa 40 Hz und etwa 200 Hz betragen.

Das Nadelmodul kann als Einwegmodul ausgeführt sein. Bei dem Nadelmodul können ein oder mehrere Bauteile aus einem Kunststoffmaterial hergestellt sein, beispielsweise mittels Spritzgießen.

Mithilfe der Hautstechvorrichtung kann ein Verfahren zum Ein- und Ausfahren einer Stechnadel ausgeführt werden. Hierbei wird die Hautstechvorrichtung mit einem Antriebsmodul und einem Nadelmodul bereitgestellt. In dem Antriebsmodul ist eine Antriebseinrichtung angeordnet, die bei dem Verfahren eine Antriebsbewegung erzeugt. Die Antriebsbewegung wird auf eine flexible Stechnadel eingekoppelt, die in dem Nadelmodul in einem Nadelkanal verlagerbar angeordnet ist. Hierdurch wird die Stechnadel vor und zurück bewegt, derart, dass eine Stechnadelspitze zumindest beim Vorwärtsbewegen durch eine Nadelaustrittsöffnung eines Gehäuses des Nadelmoduls austritt.

Das Vor- und Zurückbewegen kann in einem Beispiel mit einer von der Antriebseinrichtung bereitgestellten maschinengesteuerten Wiederholfrequenz ausgeführt werden.
Aufgrund eines Einstechwiderstands beim Vorwärtsbewegen der flexiblen Stechnadel knickt diese in einem Knickbereich des Nadelkanals ab. Ein geknickter Abschnitt der flexiblen Stechnadel weicht in einen Ausweichraum im Nadelkanal aus, der im Bereich einer Kanalerweiterung des Nadelkanals bereitgestellt ist.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung einer Hautstechvorrichtung mit einem Antriebsmodul und einem hieran gekoppelten Nadelmodul;
- Fig. 2: eine schematische perspektivische Darstellung eines Nadelmoduls mit Nadelmodulspitze teilweise im Schnitt;
- Fig. 3: eine schematische Darstellung einer Anordnung mit einem Nadelmodul und einer hierin aufgenommenen Stechnadel im Querschnitt;
- Fig. 4: eine schematische Darstellung der Anordnung vergleichbar Fig. 3, wobei ein Kanalabschnitt mit Wellenform vorgesehen ist;
- Fig. 5: eine schematische Darstellung der Anordnung vergleichbar Fig. 3, wobei ein Kanalabschnitt mittels eines zusätzlichen Röhrchens gebildet ist;
- Fig. 6: eine schematische Darstellung von Details eines Nadelmoduls und einer hierin aufgenommenen Stechnadel im Querschnitt, wobei in einem Knickbereich eines Nadelkanals ein Gehäusedurchbruch vorgesehen ist;
- Fig. 7: schematische Darstellungen von Details eines Nadelmoduls und einer hierin aufgenommenen Stechnadel im Querschnitt, wobei im Knickbereich des Nadelkanals eine Einstelleinrichtung vorgesehen ist; und
- Fig. 8: schematische Darstellungen der Anordnung ähnlich Fig. 7, wobei die Einstelleinrichtung verstellt ist im Vergleich zu Fig. 7.

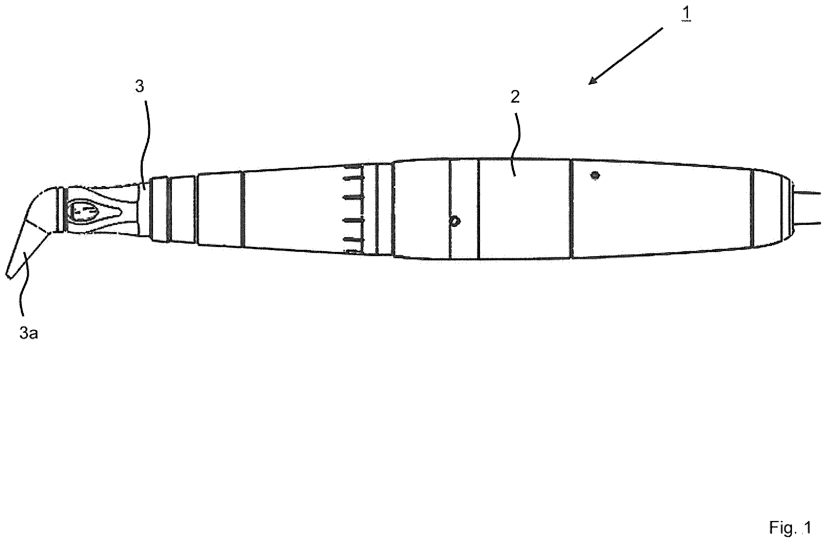

Fig. 1 zeigt eine schematische Darstellung einer Hautstechvorrichtung 1 mit einem Antriebsmodul 2 und einem hieran gekoppelten Nadelmodul 3, welches eine Nadelmodulspitze 3a aufweist.

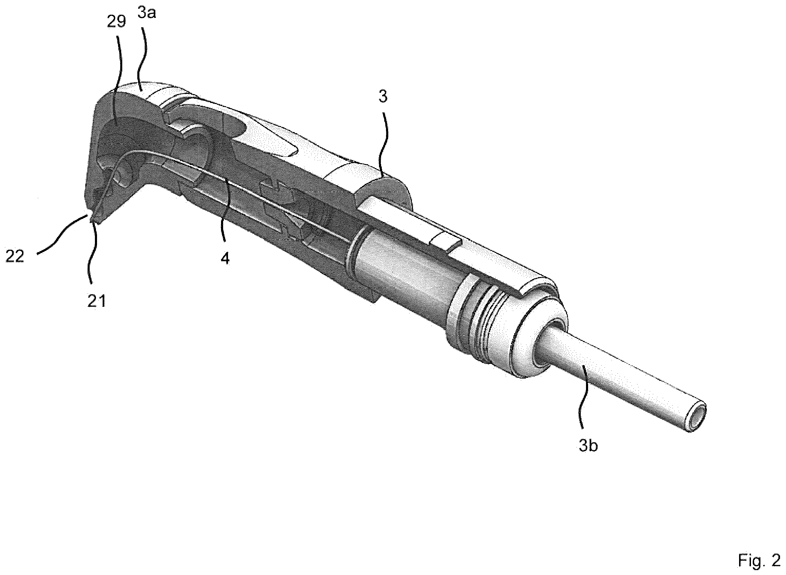

Fig. 2 zeigt eine schematische perspektivische Darstellung des Nadelmoduls 3 mit der Nadelmodulspitze 3a teilweise im Schnitt. Die Nadelmodulspitze 3a kann lösbar oder nichtlösbar am Nadelmodul 3 angeordnet sein.
Die Hautstechvorrichtung 1 ist als Handgerät ausgebildet. Mithilfe des Antriebsmoduls 2 wird eine repetierende Antriebsbewegung bereitgestellt, wie dies in verschiedenen Ausführungsformen als solches bekannt ist. Beispielsweise ist eine Antriebseinrichtung mit einem Elektromotor vorgesehen, der eine drehende Antriebsbewegung bereitstellt, welche mithilfe eines Wandlungsmechanismus in eine Vor- und Zurückbewegung gewandelt wird, die dann über ein Kopplungsbauteil 3b auf eine Stechnadel 4 eingekoppelt wird. Von der Antriebseinrichtung wird die Antriebsbewegung mit einer maschinengenerierten Wiederholfrequenz bereitgestellt.

Die Stechnadel 4 ist flexibel, derart, dass sie infolge eines Einstechwiderstands beim Aufstechen der Haut nachgeben kann und (weiter) abknickt. Die Stechnadel 4 kann in den verschiedenen Ausführungsformen als Einzelnadel oder Nadelgruppe ausgebildet sein.

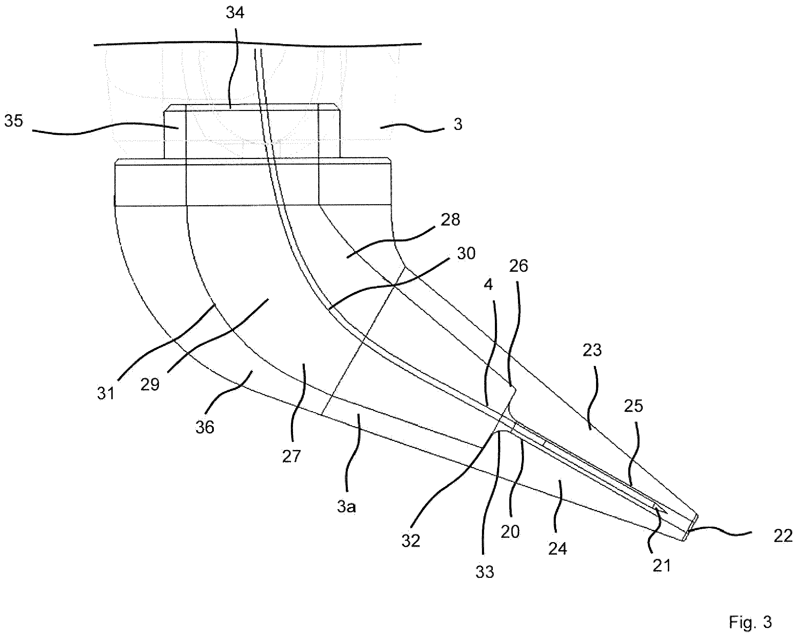

Unter Bezugnahme auf die Fig. 3 bis 8 werden nachfolgend Ausgestaltungen des Nadelmoduls 3 erläutert. Hierbei werden für gleiche Merkmale dieselben Bezugszeichen verwendet.

Fig. 3 zeigt eine schematische Darstellung einer Anordnung mit Nadelmodul 3, insbesondere mit der Nadelmodulspitze 3a und flexibler Stechnadel 4. Die flexible Stechnadel 4 ist in einem Nadelkanal 20 angeordnet, derart, dass sie infolge des Einkoppelns der Antriebsbewegung in dem Nadelkanal 20 vor und zurück bewegt werden kann, sodass im Betrieb zum lokalen Aufstechen einer Haut eine Stechnadelspitze 21 durch eine Nadelaustrittsöffnung 22 austritt, welche an der Nadelmodulspitze 3a des Nadelmoduls 3 gebildet ist. Im Bereich der Nadelmodulspitze 3a weist das Gehäuse 23 eine sich zur Nadelaustrittsöffnung 22 hin verjüngende Form auf.

Der Nadelkanal 20 weist rückseitig der Nadelaustrittsöffnung 22 einen proximalen Kanalabschnitt 25 auf. Bei der gezeigten Ausführungsform schließt sich an den proximalen Kanalabschnitt 25 rückseitig, also auf der von der Nadelaustrittsöffnung 22 abgewandten Seite, ein distaler Kanalabschnitt 26 an, der im Vergleich zum Kanalquerschnitt des proximalen Kanalabschnitts 25 mit einer Kanalerweiterung 27 gebildet ist.

Die Kanalerweiterung 27 ist insbesondere in einem Kanalknickbereich 28 des Gehäuses 23, beispielsweise in der Nadelmodulspitze 3a, und des Nadelkanals 20 ausgebildet, derart, dass ein Ausweichraum 29 in dem Nadelkanal 20 bereitgestellt ist, in welchen hinein die flexible Stechnadel 4 ausweichen kann, wenn aufgrund eines Einstechwiderstands beim Aufstechen der Haut, welcher der Bewegung der Stechnadelspitze 21 entgegenwirkt, die flexible Stechnadel 4 in einem Nadelknickbereich 30 zusätzlich abknickt und sich zur Außenseite 31 hin verlagert.

Bei der in Fig. 3 gezeigten Ausgestaltung ist der proximale Kanalabschnitt 25 mit einer geraden Rohrform gebildet. Im Übergang 32 zum distalen Kanalabschnitt 26 ist eine trichterförmige Kanalerweiterung 33 lokal ausgebildet. Hierdurch sind ein Abknicken der flexiblen Stechnadel 4 aufgrund des Einstechwiderstands sowie die Montage erleichtert.

Der Nadelkanal 20 erstreckt sich in dem Gehäuse 23 zwischen der Nadelaustrittsöffnung 22 und einer Nadeleinführöffnung 34 im Bereich eines Kopplungsabschnitts 35, mit dem die Nadelmodulspitze 3a an das restliche Gehäuse des Nadelmoduls 3 koppelt.

Das Gehäuse 23 weist, vergleichbar dem Kanalknickbereich 28, einen Gehäuseknickbereich 36 auf.

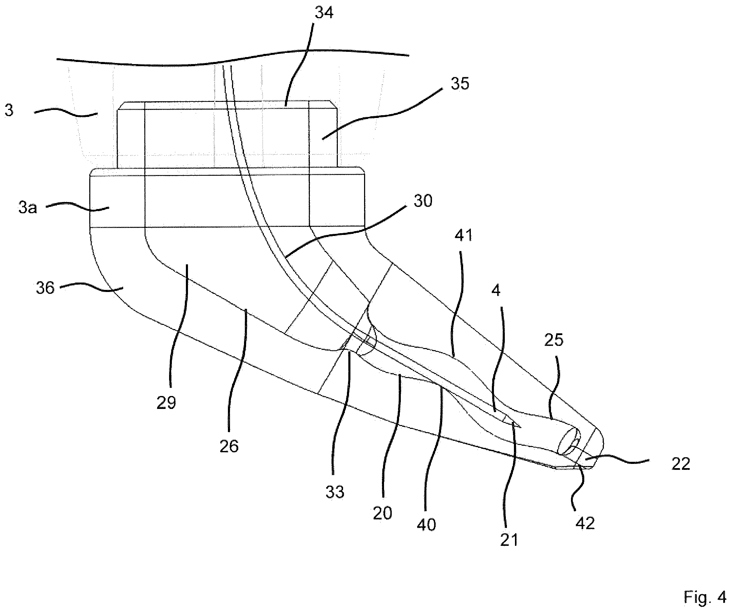

Fig. 4 zeigt einen Abschnitt des Nadelmoduls 3 und der flexiblen Stechnadel 4 im Schnitt. Der proximale Kanalabschnitt 25 ist bei der gezeigten Ausführungsform mit einer Wellenform gebildet, sodass vorstehende Wandabschnitte 40 und zurückstehende Wandabschnitte 41 in dem proximalen Kanalabschnitt 25 gebildet sind. Während beim Vor- und Zurückbewegen die vorstehenden Wandabschnitte 40 mit der flexiblen Stechnadel 4 in Kontakt kommen, findet eine solche Kontaktausbildung für die zurückstehenden Wandabschnitte 41 nicht statt.

An dem Gehäuse 23 des Nadelmoduls 3 ist benachbart zur Nadelaustrittsöffnung 22 eine Auflagefläche 42 ausgebildet, mit der das Nadelmodul 3 bei der Nutzung auf die aufzustechende Hautoberfläche aufgelegt werden kann. Die Auflagefläche 42 verläuft bei der gezeigten Ausgestaltung im Wesentlichen parallel zur Öffnungsfläche der Nadeleinführöffnung 34.

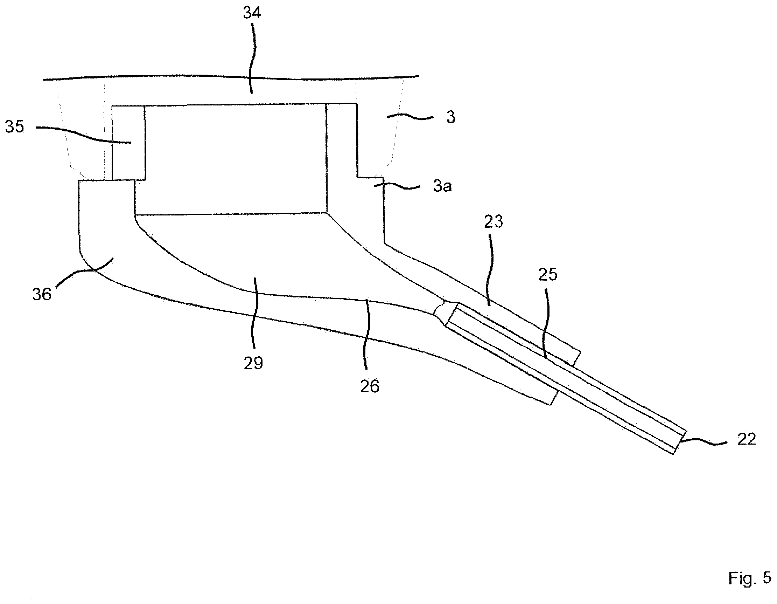

Fig. 5 zeigt eine schematische Darstellung einer weiteren Ausgestaltung des Nadelmoduls 3 im Schnitt. Die flexible Stechnadel 4 ist zur Vereinfachung nicht dargestellt.

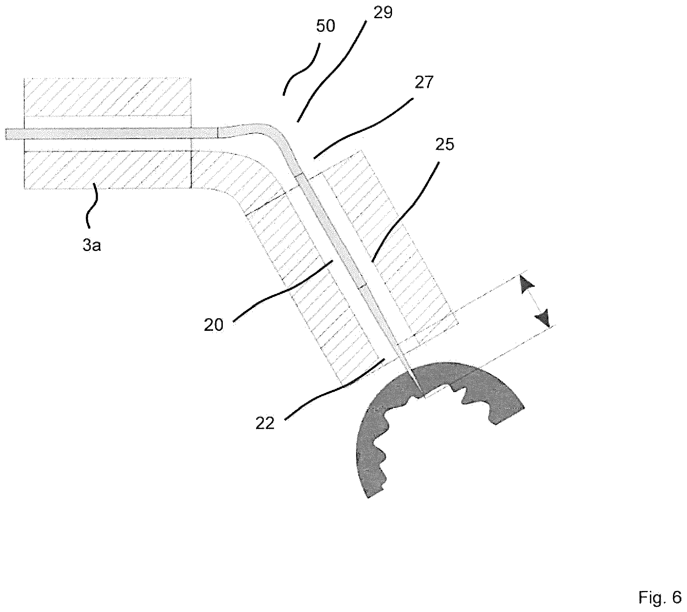

Fig. 6 zeigt eine schematische Darstellung einer anderen Ausgestaltung des Nadelmoduls 3, insbesondere der Nadelmodulspitze 3a, mit hierin aufgenommener flexibler Stechnadel 4. Im Bereich der Kanalerweiterung 27 des Nadelkanals 20 ist im Abschnitt des Ausweichraums 29 ein Gehäusedurchbruch 50 vorgesehen, in den hinein die flexible Stechnadel 4 im Fall des Einstechwiderstands abknicken kann.

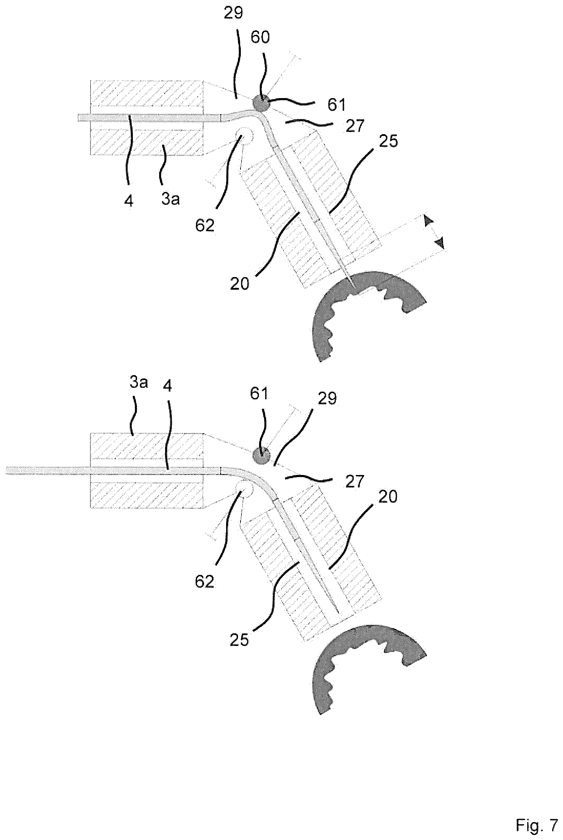

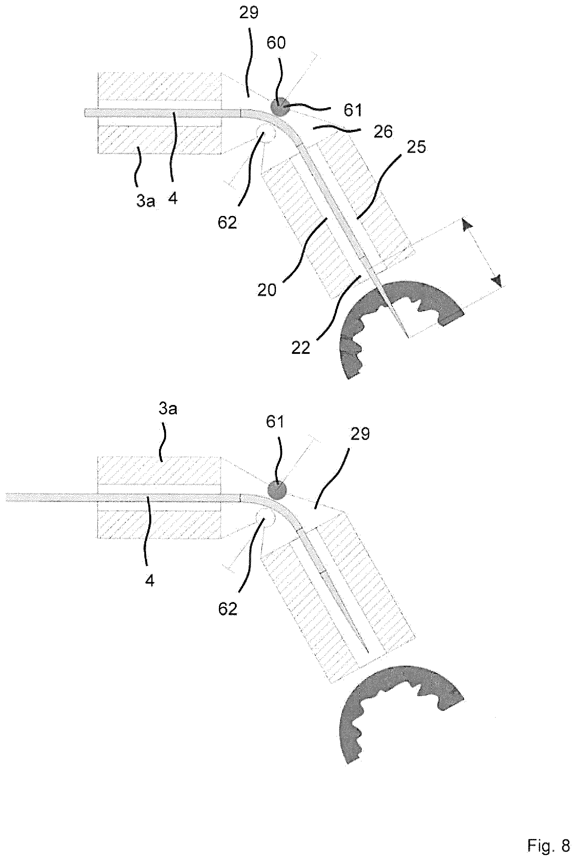

Die Fig. 7 und 8 zeigen schematische Darstellungen für weitere Beispiele des Nadelmoduls 3, insbesondere der Nadelmodulspitze 3a, und der hierin aufgenommenen flexiblen Stechnadel 4, wobei eine Einstelleinrichtung 60 im Bereich der Kanalerweiterung 27 vorgesehen und eingerichtet ist, den Ausweichraum 29 für die flexible Stechnadel 4 einzustellen. Gemäß der oberen Darstellung in Fig. 7 kann die flexible Stechnadel 4 beim Ausfahren und hierbei auftretendem Einstechwiderstand zu einem äußeren Anschlag 61 hin in den Ausweichraum 29 abknicken und wird gemäß der unteren Darstellung in Fig. 7 beim Einfahren an einem inneren Anschlag 62 geführt.

Eine veränderte Einstellung der Einstelleinrichtung 60 gemäß Fig. 8 bewirkt eine engere Führung der flexiblen Stechnadel 4 beim Aus- und Einfahren, wobei in einer Einstellung das Ausweichen in den Ausweichraum 29 im Wesentlichen ganz unterbunden sein kann.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Nadelmodul für eine Hautstechvorrichtung zum lokalen Aufstechen einer menschlichen oder tierischen Haut in Schrägrichtung, mit:
- einem Gehäuse (23) mit einer Nadelmodulspitze (3a);
- einer Nadelaustrittsöffnung (22), die an der Nadelmodulspitze (3a) frontseitig angeordnet ist;
- einer Nadeleinführöffnung (34), die an der Nadelmodulspitze (3a) rückseitig angeordnet ist;
- einem Nadelkanal (20), welcher sich in der Nadelmodulspitze (3a) zwischen der Nadeleinführöffnung (34) und der Nadelaustrittsöffnung (22) erstreckt und eingerichtet ist, eine flexible Stechnadel (4) nadelführend aufzunehmen;
- einem in Bezug auf die Nadelaustrittsöffnung (22) proximalen Kanalabschnitt (25) des Nadelkanals (20), welcher sich rückseitig der Nadelaustrittsöffnung (22) erstreckt und in welchem eine die flexible Stechnadel (4) zur Nadelaustrittsöffnung (22) hin führende proximale Nadelführung gebildet ist; und
- einem in Bezug auf die Nadelaustrittsöffnung (22) distalen Kanalabschnitt (26) des Nadelkanals (20), welcher sich rückseitig des proximalen Kanalabschnitts (25) erstreckt und in welchem der Nadelkanal (20) abgeknickt ist;
**dadurch gekennzeichnet, dass** der Nadelkanal (20) in einem Knickbereich (28) des distalen Kanalabschnitts (26) eine Kanalerweiterung (27) aufweist, bei der der Nadelkanal (20) im Knickbereich (28) mit einem Kanalquerschnitt gebildet ist, welcher größer ist als ein Kanalquerschnitt in dem proximalen Kanalabschnitt (25), derart, dass ein Ausweichraum (29) für die flexible Stechnadel (4) bereitgestellt ist, in welchen hinein die flexible Stechnadel (4) aufgrund eines Einstechwiderstands mittels Abknicken ausweichen kann.

2. Nadelmodul nach Anspruch 1, **dadurch gekennzeichnet, dass** der Ausweichraum (29) auf einer Außenseite (31) des Knickbereichs (28) ausgebildet ist.

3. Nadelmodul nach Anspruch 1 oder 2, **dadurch gekennzeichnet, dass** der Kanalquerschnitt in dem distalen Kanalabschnitt (26), ausgehend von dem proximalen Kanalabschnitt (25), sich zunehmend erweiternd ausgeführt ist.

4. Nadelmodul nach mindestens einem der vorangehenden Ansprüche, **dadurch gekennzeichnet, dass** zwischen einer Führrichtung für die flexible Stechnadel (4) in dem proximalen Kanalabschnitt (25) und der Flächennormalen auf einer Öffnungsfläche der Nadeleinführöffnung (34) ein Winkel zwischen etwa 15 Grad und etwa 90 Grad ausgebildet ist.

5. Nadelmodul nach mindestens einem der vorangehenden Ansprüche, **dadurch gekennzeichnet, dass** das Gehäuse (23) im Bereich des Ausweichraums (29) einen den Ausweichraum (29) erweiternden Durchbruch (50) aufweist.

6. Nadelmodul nach mindestens einem der vorangehenden Ansprüche, **dadurch gekennzeichnet, dass** die Kanalerweiterung (27) zwischen einem rückseitigen Ausgang des proximalen Kanalabschnitts (25) und der Nadeleinführöffnung (34) den Nadelkanal (20) zumindest teilweise erfassend gebildet ist.

7. Nadelmodul nach mindestens einem der vorangehenden Ansprüche, **dadurch gekennzeichnet, dass** an dem Gehäuse (23) eine Auflagefläche (42) angeordnet ist, die zur Öffnungsfläche der Nadelaustrittsöffnung (22) schräg gestellt ist.

8. Nadelmodul nach Anspruch 7, **dadurch gekennzeichnet, dass** die Auflagefläche (42) im Wesentlichen parallel zur Öffnungsfläche der Nadeleinführöffnung (34) angeordnet ist.

9. Nadelmodul nach mindestens einem der vorangehenden Ansprüche, **dadurch gekennzeichnet, dass** im Knickbereich (28) des Nadelkanals (20) eine Einstelleinrichtung (60) angeordnet ist, mit der ein in dem Ausweichraum (29) für das Ausweichen der flexiblen Stechnadel (4) zur Verfügung stehender Raum veränderbar ist.

10. Nadelmodul nach Anspruch 9, **dadurch gekennzeichnet, dass** die Einstelleinrichtung (60) eingerichtet ist, den für das Ausweichen der flexiblen Stechnadel (4) zur Verfügung stehenden Raum auf der Außenseite (31) und / oder der Innenseite des Knickbereichs (28) des Nadelkanals (20) einzustellen.

11. Nadelmodul nach mindestens einem der vorangehenden Ansprüche, **dadurch gekennzeichnet, dass** auf der Innenseite des Knickbereichs (28) des Nadelkanals (20) ein Führungselement (62) angeordnet ist, welches eingerichtet ist, die flexible Stechnadel (4) beim Einziehen in den Nadelkanal (20) lokal zu führen.

12. Nadelmodul nach mindestens einem der vorangehenden Ansprüche, **dadurch gekennzeichnet, dass** der Nadelkanal (20) zumindest in dem proximalen Kanalabschnitt (25) vorstehende Wandabschnitte (40), an denen die flexible Stechnadel (4) im Betrieb zur Anlage kommt, und zurückstehende Wandabschnitte (41) aufweist, an denen die flexible Stechnadel (4) im Betrieb nicht zur Anlage kommt.

13. Nadelmodul nach Anspruch 12, **dadurch gekennzeichnet, dass** die vorstehenden und die zurückstehenden Wandabschnitte (40, 41) an einer Kanalwand mit Wellenoberflächenform gebildet sind.

14. Hautstechvorrichtung zum lokalen Aufstechen einer menschlichen oder tierischen Haut in Schrägrichtung, mit:
- einem Nadelmodul (3) nach mindestens einem der vorangehenden Ansprüche;
- einer flexiblen Stechnadel (4), die in dem Nadelmodul (3) aus- und einfahrbar angeordnet ist; und
- einem Antriebsmodul (2), welches an das Nadelmodul (3) koppelt, derart, dass eine von dem Antriebsmodul (2) bereitgestellte Antriebsbewegung zum Ein- und Ausfahren auf die flexible Stechnadel (4) einkoppelbar ist.

## Claims

1. Needle module for a skin puncturing device for local puncturing of human or animal skin in an inclined direction, comprising:
- a housing (23) having a needle module tip (3a);
- a needle outlet opening (22) which is arranged on the needle module tip (3a) at the front;
- a needle insertion opening (34) which is arranged on the needle module tip (3a) at the rear;
- a needle channel (20) which extends in the needle module tip (3a) between the needle insertion opening (34) and the needle outlet opening (22) and is designed to receive a flexible puncturing needle (4) in a needle-guiding manner;
- a channel portion (25) of the needle channel (20), which portion is proximal with respect to the needle outlet opening (22), extends at the rear of the needle outlet opening (22) and in which a proximal needle guide which guides the flexible puncturing needle (4) toward the needle outlet opening (22) is formed; and
- a channel portion (26) of the needle channel (20), which portion is distal with respect to the needle outlet opening (22), extends at the rear of the proximal channel portion (25) and in which the needle channel (20) is bent;
**characterized in that** the needle channel (20) has a channel widening (27) in a bent region (28) of the distal channel portion (26), in which widening the needle channel (20) is formed in the bent region (28) so as to have a channel cross section which is larger than a channel cross section in the proximal channel portion (25) such that a deflection space (29) for the flexible puncturing needle (4) is provided, into which space the flexible puncturing needle (4) can deflect by means of bending due to a puncture resistance.

2. Needle module according to claim 1, **characterized in that** the deflection space (29) is formed on an outer side (31) of the bent region (28).

3. Needle module according to either claim 1 or claim 2, **characterized in that** the channel cross section in the distal channel portion (26), starting from the proximal channel portion (25), is designed so as to increasingly widen.

4. Needle module according to at least one of the preceding claims, **characterized in that** an angle of between approximately 15 degrees and approximately 90 degrees is formed between a guide direction for the flexible puncturing needle (4) in the proximal channel portion (25) and the surface normal on an opening surface of the needle insertion opening (34).

5. Needle module according to at least one of the preceding claims, **characterized in that** the housing (23) in the region of the deflection space (29) has a break (50) which widens the deflection space (29).

6. Needle module according to at least one of the preceding claims, **characterized in that** the channel widening (27) is formed so as to at least partly catch the needle channel (20) between a rear-side exit of the proximal channel portion (25) and the needle insertion opening (34).

7. Needle module according to at least one of the preceding claims, **characterized in that** a support surface (42) is arranged on the housing (23), which support surface is inclined with respect to the opening surface of the needle outlet opening (22).

8. Needle module according to claim 7, **characterized in that** the support surface (42) is arranged substantially in parallel with the opening surface of the needle insertion opening (34).

9. Needle module according to at least one of the preceding claims, **characterized in that** an adjusting apparatus (60) is arranged in the bent region (28) of the needle channel (20), by means of which apparatus a space which is available in the deflection space (29) for deflecting the flexible puncturing needle (4) can be changed.

10. Needle module according to claim 9, **characterized in that** the adjusting apparatus (60) is designed to adjust the space which is available for deflecting the flexible puncturing needle (4) on the outer side (31) and/or on the inner side of the bent region (28) of the needle channel (20).

11. Needle module according to at least one of the preceding claims, **characterized in that** a guide element (62) is arranged on the inner side of the bent region (28) of the needle channel (20), which guide element is designed to locally guide the flexible puncturing needle (4) when it is drawn into the needle channel (20).

12. Needle module according to at least one of the preceding claims, **characterized in that** the needle channel (20), at least in the proximal channel portion (25), has projecting wall portions (40), with which the flexible puncturing needle (4) comes into contact during operation, and recessed wall portions (41), with which the flexible puncturing needle (4) does not come into contact during operation.

13. Needle module according to claim 12, **characterized in that** the projecting and the recessed wall portions (40, 41) are formed on a channel wall having a wave surface shape.

14. Skin puncturing device for local puncturing of human or animal skin in an inclined direction, comprising:
- a needle module (3) according to at least one of the preceding claims;
- a flexible puncturing needle (4) which is arranged in the needle module (3) so as to be extensible and retractable; and
- a drive module (2) which couples to the needle module (3) such that a drive movement, provided by the drive module (2), can be coupled to the flexible puncturing needle (4) in order to retract and extend.

## Revendications

1. Module d'aiguille pour un dispositif de suture de la peau destiné au perçage local d'une peau humaine ou animale dans un sens oblique, comportant :
- un boîtier (23) comportant une pointe de module d'aiguille (3a) ;
- une ouverture de sortie d'aiguille (22) disposée à l'avant de la pointe de module d'aiguille (3a) ;
- une ouverture d'insertion d'aiguille (34) disposée à l'arrière de la pointe de module d'aiguille (3a) ;
- un canal d'aiguille (20) qui s'étend dans la pointe de module d'aiguille (3a) entre l'ouverture d'insertion d'aiguille (34) et l'ouverture de sortie d'aiguille (22) et qui est agencé pour recevoir une aiguille de perçage flexible (4) de manière à guider l'aiguille ;
- une section de canal (25) du canal d'aiguille (20) proximale par rapport à l'ouverture de sortie d'aiguille (22), qui s'étend à l'arrière de l'ouverture de sortie d'aiguille (22) et dans laquelle un guide d'aiguille proximal est formé, lequel guide d'aiguille proximal guide l'aiguille de perçage flexible (4) vers l'ouverture de sortie d'aiguille (22) ; et
- une section de canal (26) du canal d'aiguille (20) distale par rapport à l'ouverture de sortie d'aiguille (22), qui s'étend vers l'arrière de la section de canal proximale (25) et dans laquelle le canal d'aiguille (20) est replié ;
**caractérisé en ce que** le canal d'aiguille (20) présente un élargissement de canal (27) dans une zone de pliage (28) de la section de canal distale (26), dans lequel le canal d'aiguille (20) est formé dans la zone de pliage (28) avec une section transversale de canal qui est supérieure à une section transversale de canal dans la section de canal proximale (25), de telle manière qu'un espace de sortie (29) soit prévu pour l'aiguille de perçage flexible (4), dans lequel l'aiguille de perçage flexible (4) peut sortir par pliage en raison d'une résistance à la pénétration.

2. Module d'aiguille selon la revendication 1, **caractérisé en ce que** l'espace de sortie (29) est formé sur un côté extérieur (31) de la zone de pliage (28).

3. Module d'aiguille selon la revendication 1 ou 2, **caractérisé en ce que** la section transversale du canal dans la section distale du canal (26) est conçue pour s'élargir de plus en plus à partir de la section proximale du canal (25).

4. Module d'aiguille selon au moins l'une des revendications précédentes, **caractérisé en ce qu'**un angle compris entre environ 15 et environ 90 degrés est formé entre un sens de guidage de l'aiguille de perçage flexible (4) dans la section de canal proximale (25) et la normale à la surface sur une surface d'ouverture de l'ouverture d'insertion d'aiguille (34).

5. Module d'aiguille selon au moins l'une des revendications précédentes, **caractérisé en ce que** le boîtier (23) présente une ouverture (50) dans la zone de l'espace de sortie (29) qui élargit l'espace de sortie (29).

6. Module d'aiguille selon au moins l'une des revendications précédentes, **caractérisé en ce que** l'élargissement de canal (27) entre une sortie arrière de la section proximale du canal (25) et l'ouverture d'insertion d'aiguille (34) est formé de manière à venir en prise au moins partiellement dans le canal d'aiguille (20).

7. Module d'aiguille selon au moins l'une des revendications précédentes, **caractérisé en ce qu'**une surface d'appui (42) est disposée sur le boîtier (23), laquelle surface d'appui est inclinée par rapport à la surface d'ouverture de l'ouverture de sortie d'aiguille (22).

8. Module d'aiguille selon la revendication 7, **caractérisé en ce que** la surface d'appui (42) est disposée de manière sensiblement parallèle à la surface d'ouverture de l'ouverture d'insertion d'aiguille (34).

9. Module d'aiguille selon au moins l'une des revendications précédentes, **caractérisé en ce qu'**un dispositif de réglage (60) est disposé dans la zone de pliage (28) du canal d'aiguille (20), un espace disponible dans l'espace d'évitement (29) pour la sortie de l'aiguille de perçage flexible (4) pouvant être changé au moyen dudit dispositif de réglage.

10. Module d'aiguille selon la revendication 9, **caractérisé en ce que** le dispositif de réglage (60) est agencé pour ajuster l'espace disponible pour la sortie de l'aiguille de perçage flexible (4) à l'extérieur (31) et/ou à l'intérieur de la zone de pliage (28) du canal d'aiguille (20).

11. Module d'aiguille selon au moins l'une des revendications précédentes, **caractérisé en ce qu'**un élément de guidage (62) est disposé à l'intérieur de la zone de pliage (28) du canal d'aiguille (20), lequel élément de guidage est agencé pour guider localement l'aiguille de perçage flexible (4) lors du tirage de celle-ci dans le canal d'aiguille (20).

12. Module d'aiguille selon au moins l'une des revendications précédentes, **caractérisé en ce que** le canal d'aiguille (20), au moins dans la section proximale du canal (25), comporte des sections de paroi en saillie (40), contre lesquelles l'aiguille de perçage flexible (4) vient s'appuyer lors du fonctionnement, et des sections de paroi en retrait (41), contre lesquelles l'aiguille de perçage flexible (4) ne vient pas s'appuyer lors du fonctionnement.

13. Module d'aiguille selon la revendication 12, **caractérisé en ce que** les sections de paroi en saillie et en retrait (40, 41) sont formées sur une paroi de canal présentant une forme de surface ondulée.

14. Dispositif de suture de la peau destiné au perçage local d'une peau humaine ou animale dans un sens oblique, comportant :
- un module d'aiguille (3) selon au moins l'une des revendications précédentes ;
- une aiguille de perçage flexible (4) disposée dans le module d'aiguille (3) de manière déployable et rétractable ; et
- un module d'entraînement (2) qui s'accouple au module d'aiguille (3) de telle manière qu'un mouvement d'entraînement fourni par le module d'entraînement (2) peut être couplé à l'aiguille de perçage flexible (4) pour la rétraction et le déploiement.
